# EUROPEAN PATENT APPLICATION

(11) **EP 3 719 806 A1**
(43) Date of publication of application: **07.10.2020**
(21) Application number: 19167237.7
(22) Date of filing: 04.04.2019
(51) Int. Cl.: G16H 20/70, G16H 50/70

(54) **A COMPUTER-IMPLEMENTED METHOD, AN APPARATUS AND A COMPUTER PROGRAM PRODUCT FOR ASSESSING PERFORMANCE OF A SUBJECT IN A COGNITIVE FUNCTION TEST**

(71) Applicant: Koninklijke Philips N.V., 5656 AG Eindhoven (NL)
(72) Inventor: SCHUIJERS, Erik Gosuinus Petrus, 5656 AE Eindhoven (NL); KLOOSTERMAN, Herman, 5656 AE Eindhoven (NL); VOGT, Juergen, 5656 AE Eindhoven (NL); GILLIES, Murray Fulton, 5656 AE Eindhoven (NL); WEDA, Johannes, 5656 AE Eindhoven (NL); SCHMAND, Ben, 5656 AE Eindhoven (NL)
(74) Representative: de Haan, Poul Erik

(57) **Abstract**

According to an aspect, there is provided a computer-implemented method of assessing performance of a cognitive function test performed by a subject, wherein the cognitive function test requires the subject to reproduce on a testing device a reference image that comprises a plurality of segments to produce a reproduced image: obtaining data for the reproduced image, the data comprising a first time series of inputs to the testing device by the subject in reproducing the reference image; associating each input in the first time series of inputs with a respective segment in the reference image; based on the segment associated with each input, forming a second time series that indicates an order in which segments in the reproduced image were produced by the subject; analyzing the second time series to determine at least one performance measure for the subject in performing the cognitive function test; and outputting a signal representing the determined at least one performance measure.

## Description

### FIELD OF THE INVENTION

The disclosure relates to assessment of the performance of a subject in a cognitive function test, and in particular to a computer-implemented method, apparatus and computer program product for assessing the performance of the subject in a cognitive function test in which the subject is to reproduce a reference image.

### BACKGROUND OF THE INVENTION

Clinical practice often requires the determination of the mental and/or physical capability of a subject (a person, an individual, a patient) such as to support a healthcare professional in the determination of a diagnostic or a rehabilitation plan of said person for instance.

Clinical neuropsychology is the field of determining the cognitive functioning of a person by requiring them to perform various validated neuropsychological tests and, for example, scoring their ability to perform these tests against norm scores (or normative data) that are representative for healthy individuals of a comparable demographic.

Assessing cognitive functioning is relevant for various patient groups including those suffering from dementia, stroke, traumatic brain injury, multiple sclerosis, depression, epilepsy and schizophrenia, as well as for screening healthy populations. Due to the ageing population and the rapidly increasing prevalence of some of these diseases, there is an increasing recognition of the importance of cognitive functioning and that it is necessary to be able to perform a neuropsychological assessment in an efficient and reproducible manner.

One particular category of tests that can be used to assess cognitive function involve a subject drawing a picture, either by copying another picture, or by recalling the content of the picture from memory. The Rey-Osterrieth Complex Figure (ROCF) test is an example of a test that involves both of these activities, and is a neuropsychological test which is often used to probe the functioning of a variety of cognitive domains. The ROCF test is often used when assessing people with a suspected or known brain disease or brain injury as well as when assessing the cognitive development of children. The ROCF has been initially developed by Swiss psychologist Andre Rey in 1941 (Rey, A. (1941). "L'examen psychologique dans les cas d'encephalopathie traumatique.(Les problems.)". Archives de Psychologie. 28: 215-285) and further standardized by Paul-Alexandre Osterrieth in 1944 (Osterrieth, P.A. (1944). "test de copie d'une figure complex: Contribution a l'étude de la perception et de la mémoire ". Archives de Psychologie. 30: 286-356.

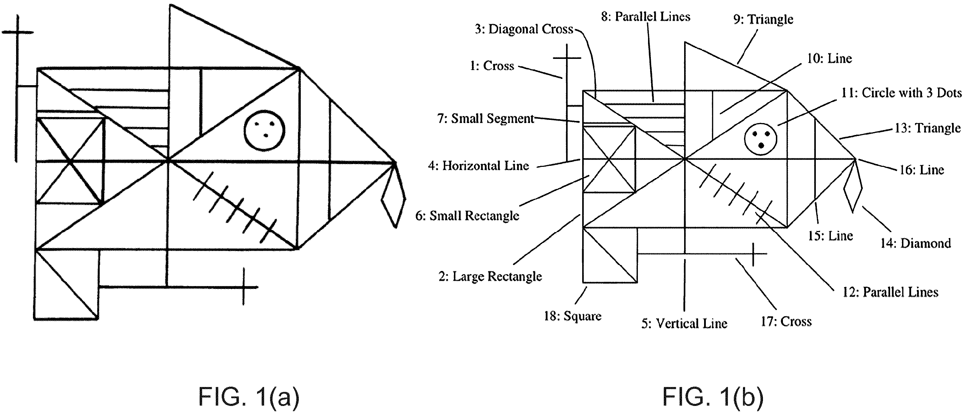

The ROCF test typically consists of three stages. In a first stage, the so-called 'copy condition', the ROCF is shown to the subject (e.g. on paper). The ROCF that is presented to the subject is shown in Fig. 1(a). The ROCF is conceptually divided into eighteen segments/elements, as shown in Fig. 1(b), along with the typical labels or names for those segments/elements. In some cases a segment or element is a single line (e.g. segment 10 - 'line'), whereas in other cases a segment or element can be more complex shape requiring the subject to draw multiple lines or components (e.g. segment 8 - 'parallel lines', and segment 11 - 'circle with 3 dots'). The subject is then requested to reproduce (copy) the ROCF as well as possible. Once the subject indicates that the copy is finished, both the original of the ROCF and the copy produced by the subject are removed from the subject's view. After a short delay, in the second stage, the so-called 'immediate recall condition', the subject is asked to reproduce the ROCF from memory. Again, after the subject indicates that the 'recall copy' is finished, it is removed from the subject's view. After a longer delay, in the third stage, the so-called 'delayed recall condition', the subject is once again asked to reproduce the ROCF from memory. During the ROCF test, the subject is not informed that they will later have to recall the ROCF from memory.

After all three stages have been completed, each of the three copies is scored. The Rey-Osterrieth score generally consists of eighteen sub-scores, one for each segment/element of the ROCF. Each segment/element is scored on accuracy and placement, with a maximum of two points per segment. More specifically, the score for a ROCF test (and similar tests) can include one or more product score(s), which is number of correctly reproduced segments, and one or more strategy score(s), which is related to the order in which segments were drawn. A score for the copy phase (the product score) quantifies visuospatial constructive ability, a ratio of the recall (product) score to the copy (product) score quantifies visuospatial memory, the strategy score quantifies organizational capacity, i.e. an aspect of executive functioning. Disadvantages of the standard scoring mechanism originally developed by Paul-Alexandre Osterrieth is that it is difficult and laborious to score the test accurately and consistently across different subjects and it provides little insight into a third aspect of performance, namely organizational strategy. Therefore, a number of additional qualitative scoring mechanisms for the ROCF test have been developed. Two examples of such qualitative scoring mechanisms are the Q-score as described in "A brief, reliable approach to coding how subjects copy the Rey-Osterrieth Complex Figure" by Bylsma, F. W., Bobholz, J. H., Schretlen, D., & Correa, D. D. (1995), Journal of the International Neuropsychological Society, Determinants of performance on the Rey-Osterrieth Complex Figure Test: An analysis, and a new technique for single-case assessment" by Bennett-Levy, J. (1984), British Journal of Clinical Psychology, 23, 109-119. It is considered that such scoring mechanisms may provide stronger differentiation between control groups and patient groups than the traditional scoring mechanism. Furthermore it may be possible to distinguish between various brain diseases, for example between (early) frontotemporal dementia and (early) Alzheimer's disease.

Visuospatial abilities, memory, attention, planning and working memory are all required for the successful completion of the ROCF, and it has been found that performing the ROCF requires a number of different cognitive domains that are not independent of each other. In particular it has been found that the organizational strategy used to produce the copy affected later memory performance. Similarly, it has been found that providing a subject with an organized approach to produce the copy resulted in a better recall performance than providing subjects with a disorganized approach. This was confirmed in "A Comparison of Qualitative Scoring Systems for the Rey-Osterrieth Complex Figure Test" by Troyer, A.K., H.A. Wishart, The Clinical Neuropsychologist, 1997, vol. 11, no. 4, pp. 381-390, which reported small correlations of 0.36 for the Q-score and a correlation of 0.34 for the Bennett-Levy mechanism to the ratio of the recall and copy accuracy scores.

### SUMMARY OF THE INVENTION

Therefore, capturing aspects of how effectively/efficiently a subject has encoded the information of the ROCF is of relevance to the neuropsychologist, not only for assessing the organizational strategy used, but also for interpreting the subsequent score obtained in the recall phase(s). In fact, if this relationship is well understood and highly correlated then it may become relevant to introduce nested norm scores, i.e. given a certain level of organization in the copy, then the interpretation of the score obtained in the recall part(s) of the test may be adjusted. While the weak correlations as reported in the paper by Troyer et al. above are insufficient for such an approach, a new measure that shows a stronger correlation to the ratio score might enable this.

Therefore the current scoring systems for the ROCF test, and similar 'copy' tests (where the subject either has to directly copy a picture or recall a picture from memory), have several difficulties in that they are difficult and laborious to score, taking a lot of time by the assessor (neuropsychologist), and that they show only a limited correlation with performance on the recall phases of the ROCF test. It is considered that the difference between the actual difference or ratio and the predicted difference or ratio may be indicative of other cognitive parameters, such as attention or visuospatial ability. The main distinction that could be made might be between dysexecutive problems and visuospatial constructive/problems. If the order in which a subject draws the segments/elements is unusual, this may be due to impairment of executive functioning. If the order is more or less normal, but the placement in space is incorrect, then this may be due to visuospatial impairments. Therefore, there is a need for improvements in the assessment of the performance of a subject in a cognitive function test, such as the ROCF test, to automate and/or standardize the scoring/assessment. For example, it is desirable to provide an indication of organizational strategy in completing one part of a test (e.g. the copy phase) that might correlate with performance in a subsequent phase of the test (e.g. the recall phase).

According to a first aspect, there is provided a computer-implemented method of assessing performance of a cognitive function test performed by a subject. In the cognitive function test the subject is to reproduce a reference image that comprises a plurality of segments to produce a reproduced image. The method comprises: obtaining data for the reproduced image, the data comprising a first time series of inputs to a testing device by the subject in reproducing the reference image; associating each input in the first time series of inputs with a respective segment in the reference image; based on the segment associated with each input, forming a second time series that indicates an order in which segments in the reproduced image were produced by the subject; analyzing the second time series to determine at least one performance measure for the subject in performing the cognitive function test; and outputting a signal representing the determined at least one performance measure. Thus the method enables the scoring/assessment of the performance of a subject in a cognitive function test, such as the ROCF test, to be automated and/or standardized.

In some embodiments the step of analyzing the second time series comprises: determining a number of transitions between segments in the second time series; and the at least one performance measure is based on the determined number of transitions. In these embodiments the at least one performance measure can include a ratio of the determined number of transitions to a predetermined minimum number of transitions to produce the reproduced image. Both of these embodiments have the advantage that the performance measure provides an indication of the organizational strategy used to produce the reproduced image. In these embodiments the at least one performance measure can also include a speed at which each segment in the reproduced image was produced.

In some embodiments the step of associating comprises: for each input in the first time series, estimating a respective probability of the input being associated with each of the plurality of segments in the reference image; and associating each input in the first time series of inputs with a respective segment in the reference image based on the estimated probabilities. These embodiments enable the segment that the input is most likely to be associated with to be identified. In these embodiments the step of estimating a probability can comprise, for each input, using a machine learning algorithm to estimate the respective probability of the input being associated with each of the plurality of segments in the reference image.

In alternative embodiments, the step of associating comprises: for a first segment in the reference image, estimating the inputs in the first time series of inputs corresponding to the first segment; and, for a second segment in the reference image, estimating the inputs in the first time series of inputs corresponding to the second segment, excluding the inputs in the first time series of inputs estimated to correspond to the first segment. These embodiments have the advantage that the computational efficiency of the associating step is improved, since the pool of available inputs reduces as inputs are associated with segments.

In other alternative embodiments, the step of associating comprises: initializing a set of vertices corresponding to inputs in the first time series and a set of connections between the inputs in the first time series; fitting the set of vertices and set of connections to the segments in the reference image according to an optimization criterion; and associating each input in the first time series with a respective segment in the reference image based on the fitted vertices and connections.

In some embodiments the method further comprises: prior to the step of analyzing, processing the second time series to identify sequences of one or more consecutive inputs that are associated with the same segment; and filtering the second time series to remove or replace any identified sequence of consecutive inputs having less than a threshold number of consecutive inputs in the sequence and/or covering less than a minimum time duration; and the step of analyzing comprises analyzing the filtered second time series to determine the at least one performance measure. These embodiments have the advantage that spurious or incorrect associations of inputs and segments, for example when a line of inputs that the subject intends to relate to one particular segment crosses a line relating to another segment that has been drawn earlier, can be removed from the time series.

In some embodiments the cognitive function test is a Rey-Osterrieth Complex Figure (ROCF) test and the reference image comprises eighteen segments.

In some embodiments, in the cognitive function test the subject is to copy the reference image to produce the reproduced image, and the method further comprises: using the determined at least one performance measure to predict a second performance measure for the subject in performing a part of the cognitive function test in which the subject is to recall the reference image from memory. These embodiments have the advantage that it may not be necessary for a subject to perform the recall phase of the cognitive function test.

In some embodiments, in the cognitive function test the subject is to recall the reference image from memory to produce the reproduced image.

According to a second aspect, there is provided a computer program product comprising a computer readable medium having computer readable code embodied therein, the computer readable code being configured such that, on execution by a suitable computer or processor, the computer or processor is caused to perform the method according to the first aspect or any embodiment thereof.

According to a third aspect, there is provided an apparatus for assessing performance of a cognitive function test performed by a subject. In the cognitive function test the subject is to reproduce a reference image that comprises a plurality of segments to produce a reproduced image. The apparatus comprises a processing unit configured to: obtain data for the reproduced image, the data comprising a first time series of inputs to a testing device by the subject in reproducing the reference image; associate each input in the first time series of inputs with a respective segment in the reference image; based on the segment associated with each input, form a second time series that indicates an order in which segments in the reproduced image were produced by the subject; analyze the second time series to determine at least one performance measure for the subject in performing the cognitive function test; and output a signal representing the determined at least one performance measure. Thus the apparatus enables the scoring/assessment of the performance of a subject in a cognitive function test, such as the ROCF test, to be automated and/or standardized.

In some embodiments the processing unit is configured to analyze the second time series by: determining a number of transitions between segments in the second time series; and the at least one performance measure is based on the determined number of transitions. In these embodiments the at least one performance measure can include a ratio of the determined number of transitions to a predetermined minimum number of transitions to produce the reproduced image. Both of these embodiments have the advantage that the performance measure provides an indication of the organizational strategy used to produce the reproduced image. In these embodiments the at least one performance measure can also include a speed at which each segment in the reproduced image was produced.

In some embodiments the processing unit is configured to associate by: for each input in the first time series, estimating a respective probability of the input being associated with each of the plurality of segments in the reference image; and associating each input in the first time series of inputs with a respective segment in the reference image based on the estimated probabilities. These embodiments enable the segment that the input is most likely to be associated with to be identified. In these embodiments the processing unit can be configured to estimate a probability by, for each input, using a machine learning algorithm to estimate the respective probability of the input being associated with each of the plurality of segments in the reference image.

In alternative embodiments, the processing unit is configured to associate by: for a first segment in the reference image, estimating the inputs in the first time series of inputs corresponding to the first segment; and, for a second segment in the reference image, estimating the inputs in the first time series of inputs corresponding to the second segment, excluding the inputs in the first time series of inputs estimated to correspond to the first segment. These embodiments have the advantage that the computational efficiency of the associating by the processing unit is improved, since the pool of available inputs reduces as inputs are associated with segments.

In other alternative embodiments, the processing unit is configured to associate by: initializing a set of vertices corresponding to inputs in the first time series and a set of connections between the inputs in the first time series; fitting the set of vertices and set of connections to the segments in the reference image according to an optimization criterion; and associating each input in the first time series with a respective segment in the reference image based on the fitted vertices and connections.

In some embodiments the processing unit is further configured to: prior to analyzing, process the second time series to identify sequences of one or more consecutive inputs that are associated with the same segment; and filter the second time series to remove or replace any identified sequence of consecutive inputs having less than a threshold number of consecutive inputs in the sequence and/or covering less than a minimum time duration; and the processing unit is configured to analyze the filtered second time series to determine the at least one performance measure. These embodiments have the advantage that spurious or incorrect associations of inputs and segments, for example when a line of inputs that the subject intends to relate to one particular segment crosses a line relating to another segment that has been drawn earlier, can be removed from the time series.

In some embodiments the cognitive function test is a Rey-Osterrieth Complex Figure (ROCF) test and the reference image comprises eighteen segments.

In some embodiments, in the cognitive function test the subject is to copy the reference image to produce the reproduced image, and the processing unit is further configured to: use the determined at least one performance measure to predict a second performance measure for the subject in performing a part of the cognitive function test in which the subject is to recall the reference image from memory. These embodiments have the advantage that it may not be necessary for a subject to perform the recall phase of the cognitive function test.

In some embodiments, in the cognitive function test the subject is to recall the reference image from memory to produce the reproduced image.

According to a fourth aspect, there is provided a system, comprising an apparatus according to the third aspect or any embodiment thereof; and a testing device for receiving the data for the reproduced image from the subject, the data comprising a first time series of inputs to the testing device by the subject.

In some embodiments the apparatus is part of the testing device. In alternative embodiments, the apparatus is separate from the testing device.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1(a) is an illustration of the Rey-Osterrieth Complex Figure (ROCF) and Fig. 1(b) is an illustration of the ROCF with the segments labelled;
Fig. 2 is a block diagram illustrating an apparatus according to an exemplary embodiment;
Fig. 3 is a flow chart illustrating a method according to an exemplary embodiment;
Fig. 4 shows an image produced by a subject in performing a cognitive function test;
Figs. 5(a)-(c) show an exemplary association of inputs in the reproduced image of Fig. 4 to segments in the ROCF; and
Fig. 6(a) is a graph illustrating a Q-score versus a ratio of a copy phase score to a recall phase score and Fig. 6(b) is a graph illustrating a performance measure determined according to an embodiment versus a ratio of a copy phase score to a recall phase score.

### DETAILED DESCRIPTION OF EMBODIMENTS

As outlined above, when a subject performs a recall phase of a test such as the ROCF test, it would be useful if the outcome (e.g. score) from the test provided a pure measure of spatial memory, as this is what the ROCF test aims to test. However, it has been shown that the performance in the recall phase provides not only a measure of spatial memory, but the outcome is also correlated to the way that the copy phase of the ROCF was performed. For example if a suboptimal strategy was used for generating the reproduced image in the copy phase, then the performance in the recall phase will be poor irrespective of the subject's spatial memory. The techniques presented herein allow for the strategy used to construct the reproduced image (e.g. the order in which segments are drawn) to be quantified in a way that the effect of this strategy on the score in the recall phase can be predicted. It therefore becomes possible to separate the contribution of a poor copy strategy on the recall phase score from the contribution of poor memory. For example the techniques presented herein enable information to be determined from a population of subjects to indicate the recall phase performances expected for certain construction strategies, and enables the performance of a subject in the recall phase to be better understood in advance (e.g. with a particular construction strategy, then a recall score of X can be expected for a healthy subject, and a recall score below this indicates that a spatial memory deficit is present.

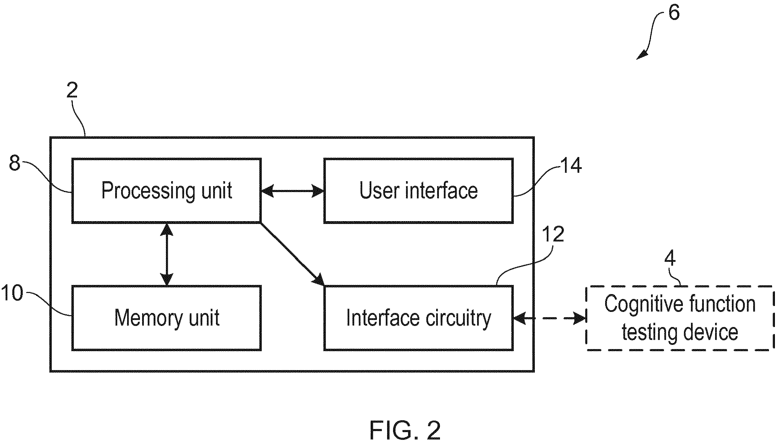

Fig. 2 shows a block diagram of an exemplary apparatus 2 that can be used for assessing performance of a subject in a cognitive function test in which the subject is to reproduce a reference image (e.g. a Rey-Osterrieth Complex Figure, ROCF), either directly by copying the reference image, or by recalling the reference image from memory. It should be noted that the 'subject' referenced herein is the person or individual that is taking part in the cognitive function test. The image produced by the subject as part of the test is referred to as a 'reproduced image', and it should be noted that it is referred to as a reproduced image regardless of whether the subject produces it as a result of directly copying the reference image (i.e. in a copy phase of the cognitive function test), or as a result of recalling the reference image from memory (i.e. in a recall phase of the cognitive function test). As used herein, a "cognitive function test" or a "test of cognitive function" can test or enable assessment of any aspect of cognitive function of a subject, including any of memory, perception, attention, motor skills, language, visual and spatial processing and executive functions.

In some embodiments, the apparatus 2 can also be used by the subject to perform or complete the cognitive function test. As such the apparatus 2 may be, or may comprise, a testing device 4 for providing the cognitive function test. Alternatively, the apparatus 2 may be configured to be connected to a separate testing device 4 that provides the cognitive function test to the subject to form a system 6 for assessing the performance of the subject in a cognitive function test. In either case, the testing device 4 may provide a user interface on which the subject can draw/produce/reproduce the reference image. The testing device 4 may also display the reference image to enable the subject to copy it during a copy phase of the test.

The testing device 4 (or the apparatus 2 in the embodiments where the apparatus 2 is used to provide the cognitive function test) can comprise a device or component that is used to store the reference image (such as the ROCF, a Taylor Figure, an image of a clock, etc.), that can output (e.g. display) the reference image to a subject or to a separate device belonging to or used by the subject (such as a computer, tablet, smartphone, television, etc.) during a copy phase of a cognitive function test, and that is used to receive and analyze the results of the inputs by the subject in producing an image. In some embodiments, the testing device 4 can be self-contained device (e.g. a computer, laptop, tablet computer, smart watch, smartphone, etc.) that stores the reference image, presents the reference image to the subject (e.g. via a display screen) during a copy phase of a cognitive function test, receives the inputs by the subject to produce the reproduced image and analyses the results to provide a measure of the performance of the subject in the test. In some other embodiments, the testing device 4 can be a device or component (e.g. a computer, server, etc.) that stores the reference image, outputs the reference image to a separate display device (e.g. a screen, a television, a smart phone, etc.), receives information representing the subject's inputs in response to the presented test and analyses the results to provide a measure of the performance of the subject in the test. In these embodiments, the testing device 4 can be a server that provides the reference image to a web browser or application on the other device, and the subject's inputs returned to the server via the web browser or application for analysis and evaluation. It will be appreciated that the above embodiments also apply where the apparatus 2 is used to provide the cognitive function tests.

The apparatus 2 is an electronic device that comprises a processing unit 8 and a memory unit 10. The processing unit 8 is configured or adapted to control the operation of the apparatus 2 and to implement the techniques described herein for assessing the performance of a subject is completing a cognitive function test.

The processing unit 8 can be configured to execute or perform the methods described herein. The processing unit 8 can be implemented in numerous ways, with software and/or hardware, to perform the various functions described herein. The processing unit 8 may comprise one or more microprocessors or digital signal processor (DSPs) that may be programmed using software or computer program code to perform the required functions and/or to control components of the processing unit 8 to effect the required functions. The processing unit 8 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

The processing unit 8 is connected to a memory unit 10 that can store data, information and/or signals for use by the processing unit 8 in controlling the operation of the apparatus 2 and/or in executing or performing the methods described herein. In some implementations the memory unit 10 stores computer-readable code that can be executed by the processing unit 8 so that the processing unit 8, in conjunction with the memory unit 10, performs one or more functions, including the methods described herein. The memory unit 10 can comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM), and electrically erasable PROM (EEPROM), and the memory unit 10 can be implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

The apparatus 2 also includes interface circuitry 12 for enabling a data connection to and/or data exchange with other devices, including any one or more of servers, databases, user devices, and one or more testing devices 4. The connection may be direct or indirect (e.g. via the Internet), and thus the interface circuitry 12 can enable a connection between the apparatus 2 and a network, such as the Internet, via any desirable wired or wireless communication protocol. For example, the interface circuitry 12 can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). In the case of a wireless connection, the interface circuitry 12 (and thus apparatus 2) may include one or more suitable antennas for transmitting/receiving over a transmission medium (e.g. the air). Alternatively, in the case of a wireless connection, the interface circuitry 12 may include means (e.g. a connector or plug) to enable the interface circuitry 12 to be connected to one or more suitable antennas external to the apparatus 2 for transmitting/receiving over a transmission medium (e.g. the air). The interface circuitry 12 is connected to the processing unit 8 to enable information or data received by the interface circuitry 12 to be provided to the processing unit 8, and/or information or data from the processing unit 8 to be transmitted by the interface circuitry 12.

In some embodiments, the apparatus 2 comprises a user interface 14 that includes one or more components that enables a user of apparatus 2 to input information, data and/or commands into the apparatus 2, and/or enables the apparatus 2 to output information or data to the user of the apparatus 2. As used herein, the 'user' of the apparatus can be a person, such as a neuropsychologist, that would like to determine a measure of the cognitive function of a subject. In embodiments where the apparatus 2 includes or is part of a testing device, the subject can also be considered as a user of the apparatus 2.

The user interface 14 can comprise any suitable input component(s), including but not limited to a keyboard, keypad, one or more buttons, switches or dials, a mouse, a track pad, a touchscreen, a stylus, a camera, a microphone, etc., and the user interface 14 can comprise any suitable output component(s), including but not limited to a display screen, one or more lights or light elements, one or more loudspeakers, a vibrating element, etc.

The apparatus 2 can be any type of electronic device or computing device. For example the apparatus 2 can be, or be part of, a server, a computer, a laptop, a tablet, a smartphone, a smartwatch, etc. In some implementations, for example where the apparatus 2 is also used as the test device to present the test(s) to the subject, the apparatus 2 can be an apparatus that is present or used in the home or care environment of the subject/user. In other implementations, the apparatus 2 is an apparatus that is remote from the subject, and remote from the home or care environment of the subject. For example, the apparatus 2 can be a server, for example a server in a data center (also referred to as being 'in the cloud').

It will be appreciated that a practical implementation of an apparatus 2 may include additional components to those shown in Fig. 2. For example the apparatus 2 may also include a power supply, such as a battery, or components for enabling the apparatus 2 to be connected to a mains power supply.

Although the exemplary methods, embodiments and techniques presented below are described with reference to the ROCF test and the ROCF shown in Fig. 1, it will be appreciated that the methods, embodiments and techniques herein are applicable to any type of cognitive function test in which a subject is to produce a reproduced image of a reference image that is comprised of multiple segments (at least conceptually, i.e. the reference image can be understood as being formed of multiple segments, even if those multiple segments, or parts of those segments, at least partially abut or overlap with each other), whether by directly copying the reference image or by recalling the reference image from memory. As such, the methods, embodiments and techniques herein are applicable to other tests including the Taylor test, which uses a different complex figure to the ROCF test, the clock drawing test in which a subject is to draw an image of an analogue clock from memory, similar tests to the clock drawing test in which another widely known object is to be drawn by the subject from memory (e.g. a bicycle, a house, a star, etc.) and the Rey Visual Design Learning Test. As noted above, a segment in the reference image can be a single line (e.g. segment 10 - 'line' in the ROCF in Fig. 1), or a more complex shape requiring the subject to draw multiple lines or components to produce it (e.g. segment 8 - 'parallel lines', and segment 11 - 'circle with 3 dots').

As noted above, there is a need for improvements in the assessment of the performance of a subject in a cognitive function test, for example the ROCF test or a Taylor test, as such tests can be difficult and laborious to score reliably and consistently, and the current scoring systems may show only a limited correlation with performance on the recall phases of the cognitive function test. Therefore it is an aim to provide a technique that enables automatic assessment of the performance of a subject (i.e. without requiring a neuropsychologist or other user to manually score the test, or be present to observe the metrics that are derived from the dynamics of creating the reproduced image). Certain embodiments have the aim of providing alternative scoring mechanisms or measures to those currently available that are more relevant for neuropsychological assessment than the current mechanisms or measures, such as alternative measures relating to organizational and/or timing-related aspects of the completion of the test by the subject. For example, it can be possible to provide an indication of organizational strategy in completing one part of the test (e.g. the copy phase) that might correlate with performance in a subsequent phase of the test (e.g. the recall phase).

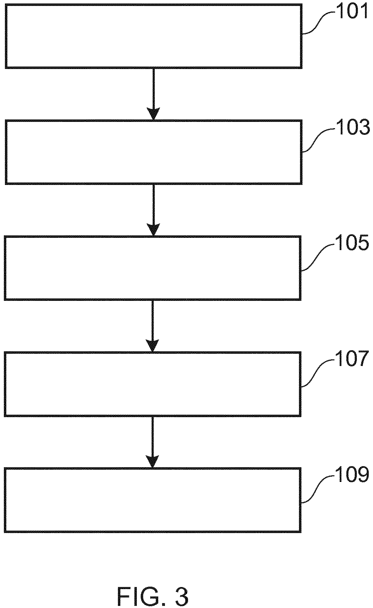

The flow chart in Fig. 3 illustrates an exemplary general method according to the techniques described herein for assessing the performance of a subject in a cognitive function test such as a ROCF test. One or more of the steps of the method can be performed by the processing unit 8 in the apparatus 2, in conjunction with any of the memory unit 10, interface circuitry 12 and user interface 14 as appropriate. The processing unit 8 may perform the one or more steps in response to executing computer program code, that can be stored on a computer readable medium, such as, for example, the memory unit 10.

In the cognitive function test, the subject provides inputs to a testing device 4 (whether a testing device 4 that is part of the apparatus 2 or a testing device 4 that is separate from the apparatus 2) to produce the reproduced image. In preferred embodiments, the inputs are provided by the subject using a touchscreen on which the subject draws using their finger(s) or a stylus. In alternative embodiments, the inputs can be provided by the subject using a stylus on an electronic drawing pad. The subject will typically produce the reproduced image by drawing a series of lines, gradually building up the reproduced image over time.

In a first step, step 101, the apparatus 2/processing unit 8 obtains data for the reproduced image produced by the subject. The data comprises a time series of inputs to the testing device 4 by the subject in reproducing the reference image to form the reproduced image. The subject may have produced the reproduced image by directly copying the reference image, or the subject can produce the reproduced image by recalling the reference image (which could be, e.g. the ROCF, a picture of an object, such as a clock, etc.) from memory.

The time series of inputs obtained in step 101 is referred to herein as a "first time series of inputs". Where the inputs are provided by the subject using a touchscreen, the data can comprise a time series of pixels on the touchscreen that have been touched by the subject or stylus in producing the reproduced image, or a time series of x-y coordinates for the positions on the touchscreen touched by the subject or stylus in producing the reproduced image. Where the inputs are provided using a stylus on an electronic drawing pad, the data can comprise a time series of x-y coordinates for the positions on the drawing pad touched by the stylus in producing the reproduced image. In some embodiments, each input in the time series of inputs can correspond to an individual line drawn by the subject using a finger or stylus (e.g. each input can be a complete pen/stylus stroke). In these embodiments, each input can be represented as a series of pixels or x-y coordinates.

In any case, the time series of inputs comprises timing information (e.g. a timestamp for each input) that indicates the order in which the subject made each input and produced the reproduced image. It will be appreciated from the above that the time series of inputs will represent the various lines drawn by the subject in producing the reproduced image, and it will be appreciated that the subject will typically draw a line, reposition the finger or stylus somewhere else on the touchscreen or drawing pad and draw another line. As such, the time series of inputs may have discontinuities in time (i.e. there can be a gap or 'no input'/'no input sample' for a time period while the finger or stylus is not touching the touchscreen or drawing pad), and/or discontinuities in space (i.e. the pixel or x-y coordinate of an input may not be adjacent to the last pixel or x-y coordinate).

The data can be obtained by the apparatus 2/processing unit 8 in step 101 in real-time or near-real-time, i.e. as the subject is producing the reproduced image on the testing device 4. Alternatively, the data can be obtained in step 101 once the subject has completed the reproduced image, for example as soon as the subject has completed the reproduced image, or by retrieving data for an earlier-completed reproduced image from the memory unit 10.

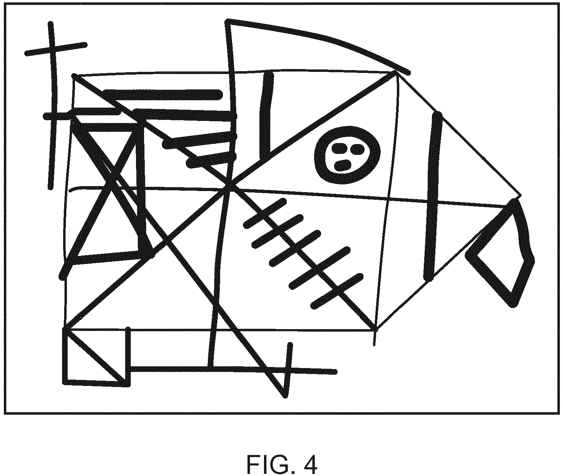

Fig. 4 shows an exemplary reproduced image produced by a subject in performing the copy phase of a ROCF test (i.e. where the subject is shown the reference image and has to copy it). The thickness of the lines shown in the reproduced image in Fig. 4 represents the relative timing of when that line was input by the subject, with the thickness of the line increasing the more recent the input. Thus, the thicker lines represent more recent inputs by the subject, whilst the thinner lines represent earlier inputs by the subject.

In step 103, the processing unit 8 associates the inputs in the time series of inputs obtained in step 101 with a respective segment in the reference image. It will be appreciated from Fig. 1(a) that the subject could reproduce the multiple segments of a complex figure like the ROCF in any particular order, with the order in which segments are reproduced varying between subjects, or between testing instances for the same subject (e.g. based on the subject being presented with different organizational strategies for reproducing the reference image). In addition, it will be appreciated that a subject may not necessarily complete a segment before moving on to another segment (not least because it may not be clear from the reference image how the reference image is divided into segments), and so different parts of a particular drawn line in the time series of inputs may in fact relate to different segments. Therefore, step 103 aims to link (associate) inputs and segments to enable a subsequent analysis of the organizational strategy used by the subject to produce the reproduced image.

In some embodiments of step 103, inputs are evaluated in turn, and a segment identified in the plurality of segments that the input is associated with. Each of the inputs can be evaluated in this way, so that each input in the time series of inputs is associated with a particular segment. This step can comprise determining a respective probability of a particular input being associated with each of the plurality of segments, and then determining which segment the input relates to based on the probability (e.g. the input can be associated with the segment that has the highest probability). In some embodiments of this step, a machine learning algorithm can be used to estimate the respective probability of the input being associated with each of the plurality of segments in the reference image. The machine learning algorithm (which will have been trained on other reproduced images produced by the subject and/or other subjects) tries to match the reproduced image to the reference image, for example by matching lines having a shape and/or position in the reproduced image to lines having a similar shape and/or position in the reference image, and/or by using the shape and position of segments in the reference image to identify corresponding segments in the reproduced image. Thus, in the case of a ROCF, the machine learning algorithm can determine, for each of the inputs in the first time series, probabilities of a particular input being associated with each of the eighteen segments.

In alternative embodiments, of step 103, a segment is evaluated individually, and all of the inputs in the first time series of inputs that form that segment in the reproduced image are identified. Each of the segments can be evaluated in this way. This step can comprise determining a respective probability of a particular input being associated with the segment, and then determining whether the input relates to the segment based on the probability (e.g. the input can be associated with the segment if the probability is above a threshold value, for example above 0.5). For a particular segment, a respective probability that the input is associated with the segment can be determined for each of the inputs. Therefore, to reduce the computational load required, in some embodiments, once an input is associated with a segment, the input is removed from the 'pool' of available inputs when the next segment is evaluated. Thus, in some embodiments, step 103 can comprise, for a first segment in the reference image, estimating the inputs in the first time series of inputs corresponding to the first segment (for example based on probabilities), and for a second segment in the reference image, estimating the inputs in the first time series of inputs corresponding to the second segment, excluding the inputs in the first time series of inputs estimated to correspond to the first segment.

In other alternative embodiments of step 103, inputs and segments can be associated with each other based on fitting of a vectorized reference image to the reproduced image. In particular, step 103 can comprise initializing a set of vertices corresponding to inputs in the first time series, and initializing a set of connections between the inputs in the first time series. The set of vertices and the set of connections can be fitted to the segments in the reference image according to an optimization criterion, and each input in the first time series can be associated with a respective segment in the reference image based on the fitted vertices and connections. With each iteration of these steps, the optimization criterion acts to fit the reproduced image to the reference image. The optimization criterion can be, for example, based on a distance between an input and a part of a segment, e.g. a summation of the shortest pixel distance to the set of segments over all pixels of the input, with an input being associated with a segment with which it has the shortest distance.

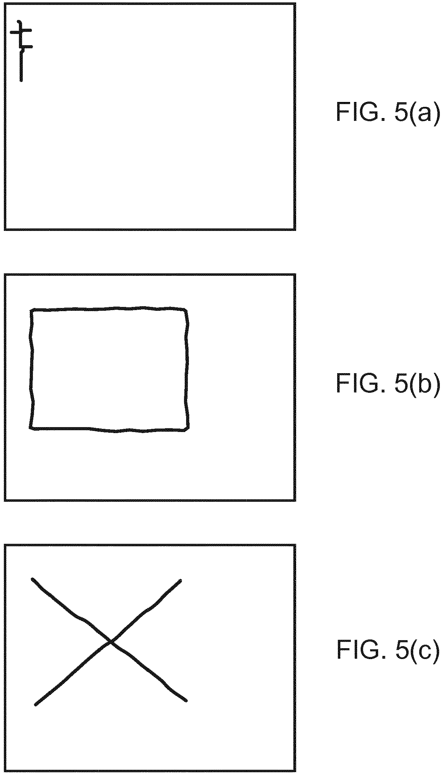

The illustrations in Figs. 5(a)-(c) show an exemplary association of inputs in the reproduced image of Fig. 4 to segments in the ROCF as a result of step 103. Fig. 5(a) shows the inputs from the reproduced image in Fig. 4 that have been associated with the cross segment (segment 1 shown in Fig. 1(b)), Fig. 5(b) shows the inputs from the reproduced image in Fig. 4 that have been associated with the large rectangle segment (segment 2 shown in Fig. 1(b)) and Fig. 5(c) shows the inputs from the reproduced image in Fig. 4 that have been associated with the diagonal cross (segment 3 shown in Fig. 1(b)).

After step 103, a second time series is formed that indicates an order in which the segments in the reference image were produced by the subject in the reproduced image (step 105). Effectively, in this step, each input in the first time series of inputs is labelled with the identity of the segment that the input has been determined to be associated with in step 103. If each segment in the reference image is assigned a unique ID, e.g. a letter (so segment 1: cross = A; segment 2: large rectangle = B; segment 3: diagonal cross = C, etc.), step 105 can result in an exemplary second time series as follows:
Second time series: AAAAABBBBBBBRRBBBBCCCCC... which indicates that the first five inputs in the first time series of inputs related to the subject drawing the cross (segment 1), the next seven inputs related to the subject drawing part of the large rectangle (segment 2), the next two inputs in the first time series of inputs related to the subject drawing the square (segment 18), the next four inputs related to the subject drawing more of the large rectangle (segment 2), the next five inputs related to the subject drawing the diagonal cross (segment 3), and so on. A change from the subject producing one segment to producing another is referred to as a 'transition'. In the exemplary second time series above, there is a transition from segment 1 (at the end of the first five inputs) to segment 2 (the next seven inputs), another transition from segment 2 (at the end of those seven inputs) to segment 18 (the next two inputs), etc.

It will be appreciated that the subject may not have completed each segment of the reference image before moving on to another segment, and this can be seen in the exemplary second time series above where the subject is deemed to have drawn part of segment 2 before drawing part of segment 18 and then drawing another part of segment 2. It will also be appreciated that the subject may not have drawn the segments according to the numbering of the segments in the reference image, and this can also be seen in the exemplary second time series above.

In practice, it may be that from examining the second time series, it is possible to determine that the second time series contains spurious or incorrect associations of inputs and segments, for example when a line of inputs that the subject intends to relate to one particular segment crosses a line relating to another segment that has been drawn earlier. For example, in the ROCF, when drawing segment 12 (parallel lines), each line will cross one of the lines in segment 3 (diagonal cross), and it is possible for the input at any crossing or overlapping point to be associated with either segment. These spurious or incorrect associations will manifest in the second time series as short (or even single) occurrences of a particular segment, and in some embodiments they can be identified and filtered or smoothed out before the subsequent steps in the method. Therefore, in some embodiments the processing unit 8 can process the second time series to identify sequences of one or more consecutive inputs that are associated with the same segment (e.g. from the above example, the sequences are AAAA, BBBBBBB, RR, BBBB, CCCCC, ...) and the second time series is filtered to remove or replace any identified sequence of consecutive inputs associated with the same segment having less than a threshold number of consecutive inputs in the sequence. Alternatively the second time series can be filtered to remove or replace any identified sequence of consecutive inputs covering less than a minimum time duration.

The filtering can comprise discarding (removing) any sequence from the second time series that has less than the threshold number of consecutive inputs or a duration less than the minimum duration. For example, in the above example, if the threshold number is 3 (or the minimum duration is roughly equal to the duration of three inputs), the RR sequence will be identified and discarded from the second time series. Alternatively the filtering can comprise replacing the segment identity associated with any sequence from the second time series that has less than the threshold number of consecutive inputs or a duration less than the minimum duration with the identity of a neighboring sequence. For example, in the above example, the RR sequence will be identified and replaced with BB, as the RR sequence is bounded by two 'B' sequences. Conceptually, the above examples can be understood as the subject having drawn segment 2 (the large rectangle), and step 103 has incorrectly associated the inputs relating to the part of the line shared by segment 2 and segment 18 (the square). By removing of replacing this misclassification, the reliability of the information represented by the second time series is improved.

The output of step 105 (either the second time series or the filtered second time series) is then analyzed to determine at least one performance measure for the subject in performing the cognitive function test (step 107). The performance measure can be based on any aspect of the second time series, for example the order in which the subject produced the segments, the time taken to produce each segment, the speed that each segment is produced, the number of transitions between producing different segments, etc.

In the case of the performance measure being based on the number of transitions, the performance measure can provide an indication of the organizational strategy that the subject used to produce the reproduced image, e.g. a higher number of transitions suggests that the subject only partially completed various segments and had to return to the segments one or more times before it was completed. A lower number of transitions (particularly in a recall phase of the cognitive function test) indicates that the subject has likely stored the reference image in their memory segment by segment, and are reproducing it according to that strategy. In some embodiments, the performance measure is based on a comparison between the number of transitions the subject took to produce the reproduced image and a minimum possible number transitions that the subject could have taken to complete the reproduced image. For example, in the case of the ROCF, the minimum number of transitions is seventeen. In some embodiments, the comparison is a ratio of the number of transitions the subject took to produce the reproduced image and the minimum possible number of transitions that the subject could have taken to complete the reproduced image. This ratio is referred to herein as a 'fragmentation score'.

The performance measure determined in step 107 is then output as a signal, for example to the user interface 14 for presentation to the subject or a user (e.g. neuropsychologist), and/or to another device or apparatus via the interface circuitry 12.

It has been found that the ratio of the number of transitions the subject took to produce the reproduced image to the minimum possible number of transitions that the subject could have taken to complete the reproduced image is approximately proportional to the copy/recall ratio that is typically used in the ROCF test (i.e. the ratio of the conventional ROCF scores in the copy and recall phases) and similar tests to assess the performance of the subject over the copy and recall phases of the test. That is, if the subject has stored the reference image in their memory segment by segment, the subject is likely to reproduce each segment completely before drawing the next one.

Therefore, where the fragmentation score is determined during a copy phase of a cognitive function test, the fragmentation score can be used to predict the difference or ratio of the copy phase score to the (delayed) recall phase score. Thus, the method can further include using the performance measure (fragmentation score) to predict a performance measure for the subject in performing a recall part of the cognitive function test.

The fragmentation score can be understood as a score related to executive function or organizational strategy/planning for the ROCF test or similar tests. Thus, in some embodiments, determining this fragmentation score may mean that the subject does not need to perform the recall phase(s) of the test.

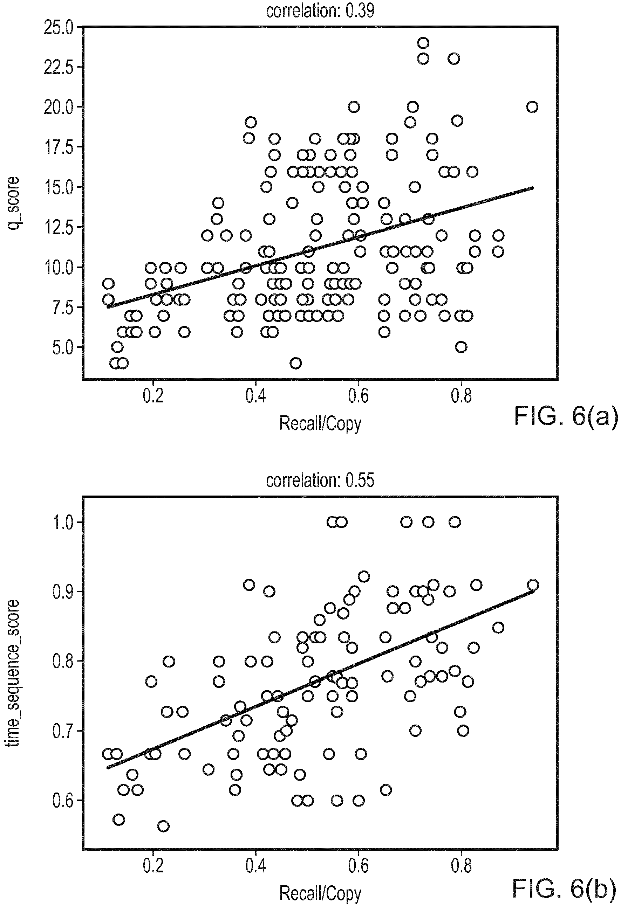

The graphs in Fig. 6 illustrate the usefulness of the fragmentation score as described in the above embodiments. Data for 100 'healthy' (i.e. no known cognitive impairment) individuals was collected using the ROCF test, and two neuropsychologists determined the Q-score for the produced complex figures (the Q-score from the neuropsychologists for each individual was averaged to determine an overall Q-score for that individual. Figure 6(a) is a graph illustrating the (overall) Q-score versus a ratio of a copy phase score to a recall phase score for those individuals, and it can be seen that there is a correlation between the Q-score and the ratio of the copy phase score to the recall phase score. However, this correlation is weak, which is comparable to that reported in "A Comparison of Qualitative Scoring Systems for the Rey-Osterrieth Complex Figure Test" by Troyer et al mentioned above. Figure 6(b) is a graph of the fragmentation score versus the ratio of the copy phase score to the recall phase score. It can be seen that the correlation between the fragmentation score and the ratio of the copy phase score to the recall phase score (correlation = 0.55) is significantly higher than the correlation of the Q-score and the ratio of the copy phase score to the recall phase score (correlation = 0.39). This shows that the fragmentation score is a better performance measure than the conventional Q-score.

Therefore there is provided an improved technique for the assessment of the performance of a subject in a cognitive function test, such as the ROCF test, and in particular enables the scoring/assessment to be automated and/or standardized, resulting in a more cost-effective and reliable outcome. In some embodiments, the performance measure provides an indication of the subject's organizational strategy in completing one part of the test (e.g. the copy phase), and that can be used to predict performance of the subject in a subsequent phase of the test (e.g. the recall phase).

While the performance measure defined in this disclosure is described as being useful for predicting the score in a recall phase, it will be appreciated that the measure can also be used to characterize how an image produced in a recall phase has been constructed. A comparison of the performance measure derived from the copy phase and the performance measure derived from the recall phase could for example give a measure of how consistent the subject is in spatial planning.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method of assessing performance of a cognitive function test performed by a subject, wherein the cognitive function test requires the subject to reproduce on a testing device a reference image that comprises a plurality of segments to produce a reproduced image, the method comprising:
obtaining data for the reproduced image, the data comprising a first time series of inputs to the testing device by the subject in reproducing the reference image;
associating each input in the first time series of inputs with a respective segment in the reference image;
based on the segment associated with each input, forming a second time series that indicates an order in which segments in the reproduced image were produced by the subject;
analyzing the second time series to determine at least one performance measure for the subject in performing the cognitive function test; and
outputting a signal representing the determined at least one performance measure.

2. A method as claimed in claim 1, wherein the step of analyzing the second time series comprises:
determining a number of transitions between segments in the second time series; wherein the at least one performance measure is based on the determined number of transitions.

3. A method as claimed in claim 2, wherein the at least one performance measure includes a ratio of the determined number of transitions to a predetermined minimum number of transitions to produce the reproduced image.

4. A method as claimed in any of claims 1-3, wherein the method further comprises:
prior to the step of analyzing, processing the second time series to identify sequences of one or more consecutive inputs that are associated with the same segment; and
filtering the second time series to remove or replace any identified sequence of consecutive inputs having less than a threshold number of consecutive inputs in the sequence and/or covering less than a minimum time duration;
wherein the step of analyzing comprises analyzing the filtered second time series to determine the at least one performance measure.

5. A method as claimed in any of claims 1-4, wherein the cognitive function test is a Rey-Osterrieth Complex Figure (ROCF) test.

6. A method as claimed in any of claims 1-5, wherein in the cognitive function test the subject is to copy the reference image to produce the reproduced image, wherein the method further comprises:
using the determined at least one performance measure to predict a second performance measure for the subject in performing a part of the cognitive function test in which the subject is to recall the reference image from memory.

7. A method as claimed in any of claims 1-5, wherein the cognitive function test requires the subject to recall the reference image from memory to produce the reproduced image.

8. A computer program product comprising a computer readable medium having computer readable code embodied therein, the computer readable code being configured such that, on execution by a suitable computer or processor, the computer or processor is caused to perform the method of any of claims 1-7.

9. An apparatus for assessing performance of a cognitive function test performed by a subject, wherein the cognitive function test requires the subject to reproduce a reference image on a testing device that comprises a plurality of segments to produce a reproduced image, the apparatus comprises a processing unit configured to:
obtain data for the reproduced image, the data comprising a first time series of inputs to the testing device by the subject in reproducing the reference image;
associate each input in the first time series of inputs with a respective segment in the reference image;
based on the segment associated with each input, form a second time series that indicates an order in which segments in the reproduced image were produced by the subject;
analyze the second time series to determine at least one performance measure for the subject in performing the cognitive function test; and
output a signal representing the determined at least one performance measure.

10. An apparatus as claimed in claim 9, wherein the processing unit is configured to analyze the second time series by:
determining a number of transitions between segments in the second time series; wherein the at least one performance measure is based on the determined number of transitions.

11. An apparatus as claimed in claim 10, wherein the at least one performance measure includes a ratio of the determined number of transitions to a predetermined minimum number of transitions to produce the reproduced image.

12. An apparatus as claimed in any of claims 9-11, wherein the processing unit is configured to:
prior to analyzing, process the second time series to identify sequences of one or more consecutive inputs that are associated with the same segment; and
filter the second time series to remove or replace any identified sequence of consecutive inputs having less than a threshold number of consecutive inputs in the sequence and/or covering less than a minimum time duration;
wherein the processing unit is configured to analyze the filtered second time series to determine the at least one performance measure.

13. An apparatus as claimed in any of claims 9-12, wherein the cognitive function test is a Rey-Osterrieth Complex Figure (ROCF) test.

14. An apparatus as claimed in any of claims 9-13, wherein in the cognitive function test the subject is to copy the reference image to produce the reproduced image, wherein the processing unit is further configured to:
use the determined at least one performance measure to predict a second performance measure for the subject in performing a part of the cognitive function test in which the subject is to recall the reference image from memory.

15. A system, comprising:
an apparatus according to any of claims 9-14; and
a testing device for receiving the data for the reproduced image from the subject, the data comprising a first time series of inputs to the testing device by the subject.
